(19) Europäisches Patentamt
European Patent Office
Office européen des brevets

(11) **EP 4 464 326 A1**

(12) **EUROPÄISCHE PATENTANMELDUNG**

(43) Veröffentlichungstag:
**20.11.2024 Patentblatt 2024/47**

(21) Anmeldenummer: 23174069.7

(22) Anmeldetag: **17.05.2023**

(51) Internationale Patentklassifikation (IPC):
**A61K 36/185** (2006.01)     **A61K 36/31** (2006.01)
**A61K 9/14** (2006.01)     **A61P 31/00** (2006.01)

(52) Gemeinsame Patentklassifikation (CPC):
(C-Sets verfügbar)
**A61P 31/00; A61K 9/14; A61K 36/185;
A61K 36/31;** A61K 2236/31; A61K 2236/33  (Forts.)

(84) Benannte Vertragsstaaten:
**AL AT BE BG CH CY CZ DE DK EE ES FI FR GB
GR HR HU IE IS IT LI LT LU LV MC ME MK MT NL
NO PL PT RO RS SE SI SK SM TR**
Benannte Erstreckungsstaaten:
**BA**
Benannte Validierungsstaaten:
**KH MA MD TN**

(71) Anmelder: **KWIZDA Pharma GmbH
1160 Wien (AT)**

(72) Erfinder:
• **WITTERNIGG, Anja
1190 Wien (AT)**
• **FEICHTINGER, Silke
1230 Wien (AT)**
• **REISS, Wolfgang
1100 Wien (AT)**

(74) Vertreter: **SONN Patentanwälte GmbH & Co KG
Riemergasse 14
1010 Wien (AT)**

(54) **HERSTELLUNG EINER PHARMAZEUTISCHEN DARREICHUNGSFORM AUS MEERRETTICH UND KAPUZINERKRESSE**

(57) Offenbart wird ein Verfahren zur Herstellung einer pharmazeutischen Darreichungsform, umfassend die folgenden Schritte: Bereitstellen einer trockenen Extraktkomponente aus Meerrettich (*Armoracia rusticana*) und Kapuzinerkresse (*Tropaeolum majus*) mit einer sehr geringen endogenen Myrosinase-Aktivität; Bereitstellen einer trockenen Drogenkomponente mit Meerrettichwurzel (*Armoraciae rusticanae* radix) und/oder Kapuzinerkressenkraut (*Tropaeoli maji* herba); Trockenmischen der Extraktkomponente mit der Drogenkomponente; und Bereitstellen der Mischung in der pharmazeutischen Darreichungsform. Ebenfalls offenbart wird eine nach diesem Verfahren erhältliche Darreichungsform.

**EP 4 464 326 A1**

(52) Gemeinsame Patentklassifikation (CPC): (Forts.)

C-Sets
**A61K 36/185, A61K 2300/00;**
**A61K 36/31, A61K 2300/00**

**Beschreibung**

Das Gebiet der vorliegenden Erfindung ist das der Verfahren zur Herstellung von pharmazeutischen Darreichungsformen aus Meerrettich (*Armoracia rusticana*) und Kapuzinerkresse *(Tropaeolum majus)* .

Der Meerrettich ist eine Pflanzenart aus der Gattung *Armoracia* innerhalb der Familie der Kreuzblütengewächse (Brassicaceae), die wiederum Mitglied der Ordnung der Kreuzblütlerartigen (Brassicales) ist. Seine Wurzel (*Armoraciae rusticanae* radix) ist seit langer Zeit in der Pflanzenheilkunde bekannt. Sie wird auch heute noch - unter anderem in Pulverform - in pflanzlichen Arzneimitteln gegen Erkältungskrankheiten und Harnwegsinfektionen verwendet.

Ebenfalls ein Vertreter der Kreuzblütlerartigen ist die Große Kapuzinerkresse (hierin wird "Kapuzinerkresse" als Synonym verwendet) aus der Gattung *Tropaeolum* innerhalb der Familie der Kapuzinerkressengewächse (Tropaeolaceae). Kapuzinerkressenkraut (*Tropaeoli maji* herba) findet seit langem Verwendung in der Pflanzenheilkunde und kommt beispielsweise bei Blasenentzündungen zum Einsatz.

Neben anderen wertvollen Inhaltsstoffen, wie beispielsweise Flavonoiden und Vitaminen, enthalten beide genannten Pflanzen Glucosinolate als pharmazeutisch bedeutsame Bestandteile. Diese Glucosinolate kommen (beispielsweise beim Zerkleinern oder Zerkauen von Pflanzenmaterial) mit dem sich in anderen Kompartimenten befindlichen Enzym Myrosinase (EC 3.2.1.147, vormals EC 3.2.1.1; auch Sinigrinase oder Thioglucosidase genannt) in Kontakt; durch die enzymatische Umsetzung entstehen eine Vielzahl von Verbindungen wie Isothiocyanate (Senföle), Nitrile, Thiocyanate, Epithionitrile und Oxazolidine. Von zentraler pharmakologischer Bedeutung sind die Isothiocyanate. Diese sind, im Gegensatz zu ihren Ausgangsstoffen, biologisch wirksam, nämlich beispielsweise antimikrobiell und krebshemmend.

Die antibakteriellen Effekte der Isothiocyanate aus Meerrettich werden beispielsweise in Negro, et al, "Phytochemical and functional analysis of horseradish (Armoracia rusticana) fermented and non-fermented root extracts." Fitoterapia 162 (2022): 105282, beschrieben. Die antibakteriellen Effekte der Isothiocyanate aus Kapuzinerkresse werden z.B. in folgender Schrift behandelt: Vrca, et al. "Chemical Composition and Biological Activity of Essential Oil and Extract from the Seeds of Tropaeolum majus L. var. altum." Food Technology and Biotechnology 60 (2022): 4.

Als traditionelles pflanzliches Arzneimittel mit Kapuzinerkressenkraut und Meerrettichwurzel ist das Präparat ANGOCIN® Anti-Infekt Uro+Grippal Filmtabletten (Pharmazeutischer Hersteller: Repha GmbH, Deutschland) in Apotheken erhältlich. Es wird eingesetzt bei Erkältungskrankheiten und grippalen Infekten sowie bei unkomplizierten Infektionen der unteren Harnwege (Zystitis). Dieses Präparat hat jedoch den Nachteil, dass bis zu 25 Filmtabletten am Tag eingenommen werden müssen - bereits die Standarddosis für einen Erwachsenen beträgt 12 Filmtabletten am Tag. Dies ist der Compliance der Patienten abträglich. Es ist daher eine Aufgabe der vorliegenden Erfindung, ein Herstellungsverfahren für ein Präparat aus Kapuzinerkressenkraut und Meerrettichwurzel, im welchem die wertvollen Inhaltsstoffe (insbesondere Glucosinolate) in konzentrierterer Form vorliegen, zur Verfügung zu stellen. Dadurch kann die Menge bzw. Anzahl der am Tag verabreichten Darreichungsformen reduziert werden. Dies bringt unter anderem die Vorteile mit sich, dass die Compliance der Patienten erhöht wird und sich der Aufwand für die Verpackung der Darreichungsformen (der aufgrund der Erfordernisse der Good Manufacturing Practice - GMP - beträchtlich sein kann) reduziert.

Die vorliegende Erfindung stellt ein Verfahren zur Herstellung einer vorzugsweise festen pharmazeutischen Darreichungsform zur Verfügung. Dieses umfasst die folgenden Schritte:

- Bereitstellen einer trockenen Extraktkomponente aus Meerrettich (insbesondere aus Meerrettichwurzel) und Kapuzinerkresse (insbesondere aus Kapuzinerkressenkraut) mit einer (endogenen) Myrosinase-Aktivität unter 0,05 U/g, bevorzugt unter 0,01 U/g, insbesondere unter 0,001 U/g;

- Bereitstellen einer trockenen Drogenkomponente mit Meerrettichwurzel und/oder Kapuzinerkressenkraut;

- Trockenmischen der Extraktkomponente mit der Drogenkomponente; und

- Bereitstellen der Mischung in der pharmazeutischen Darreichungsform.

Es versteht sich, dass dieses Verfahren weitere Schritte aufweisen kann.

Daneben stellt die Erfindung eine vorzugsweise feste pharmazeutische Darreichungsform, die nach diesem Verfahren herstellbar ist, zur Verfügung.

In einem weiteren Aspekt betrifft die Erfindung eine vorzugsweise feste pharmazeutische Darreichungsform, umfassend eine (trockene) Extraktkomponente - d.h. eine Komponente, die zumindest einen pflanzlichen Extrakt enthält - aus Meerrettich (insbesondere aus Meerrettichwurzel) und Kapuzinerkresse (insbesondere aus Kapuzinerkressenkraut) sowie eine Drogenkomponente - d.h. eine Komponente, die zumindest eine pflanzliche Droge enthält - mit Meerrettichwurzel und/oder Kapuzinerkressenkraut.

Diese Darreichungsformen können weitere Komponenten (z.B. andere, nicht-pflanzliche Wirkstoffkomponenten) enthalten. Zusätzlich oder alternativ dazu kann die Extraktkomponente weitere Bestandteile (z.B. Extrakte aus anderen Pflanzen und/oder Hilfsstoffe) enthalten bzw. kann die Drogenkomponente weitere Bestandteile (z.B. andere pflanzliche Drogen, beispielsweise in Pulverform, und/oder Hilfsstoffe) enthalten.

Im Zuge der vorliegenden Erfindung hat sich überraschenderweise herausgestellt, dass beim Herstellen eines organischen Auszugs aus Meerrettich bzw. Kapuzinerkresse (insbesondere bei einer alkoholischen Extraktion wie der methanolischen Extraktion, vor allem mit Methanol >98%) nicht genug Myrosinase-Aktivität erhalten bleibt, um die im Auszug vorhandenen Glucosinolate (im Gastrointestinaltrakt nach der Einnahme) in die biologisch wirksamen Isothiocyanate umzuwandeln. Vor diese Herausforderung gestellt, entwickelten die Erfinder die vorliegende Erfindung auf Basis der Zugabe der (Roh)droge zum Extrakt, um eine ausreichende Myrosinase-Aktivität (im Gastrointestinaltrakt nach der Einnahme) sicherzustellen. Ein besonderer Vorteil der Erfindung ist, dass durch Zugabe der Droge (anstatt beispielsweise aufgereinigte Myrosinase zuzugeben, die womöglich nicht in einer Form erhältlich ist, die der endogenen Form von Meerrettich bzw. Kapuzinerkresse entspricht und/oder höchste Produktionsstandards nicht erfüllt) das reichhaltige, ursprüngliche Wirkstoffprofil besser erhalten bleibt und sich das Endprodukt näher an der bereits jahrzehntelang erprobten, ursprünglichen Drogenkombination orientiert.

Im Stand der Technik wird die Zugabe von Myrosinase zu glucosinathaltigem Pflanzenmaterial lediglich im Allgemeinen beschrieben, so dass die Fachperson daraus keinerlei Hinweise zur erfindungsgemäßen Lösung gewinnen kann:

Die WO 2009/012485 A1 betrifft Verfahren zur Umwandlung von Glucosinolat in einem glucosinolathaltigen Pflanzenmaterial. Jenes Verfahren umfasst die folgenden Schritte: Bereitstellen einer Menge an verarbeitetem glucosinolathaltigem Pflanzenmaterial; Bereitstellen einer Menge an glucosinolatumwandelnder Enzymaktivität; Mischen des verarbeiteten Materials mit der Menge an Enzymaktivität; Hydratisieren der Mischung; und Inkubieren der hydratisierten Mischung, wobei die Glucosinolate enzymatisch umgewandelt werden. Ferner werden Herbizide, Fungizide, Insektizide, bakteriostatische oder bakterizide, kosmetische oder pharmazeutische Zusammensetzungen auf Grundlage dieses Verfahrens offenbart. Im Dokument ist jedoch keine Rede davon, dass die letzten beiden Schritte jenes Verfahrens *in vivo* durchgeführt werden können. Es wird auch keinesfalls nahegelegt, dass die hierin genannten Extrakte und Drogen gemeinsam in einer Darreichungsform vorliegen können.

Die US 9,486,434 B2 bezieht sich auf ein Verfahren zur Verabreichung von Isothiocyanaten zur Therapie von Blasenkrebs. Offenbart wird unter anderem eine Zusammensetzung, die eine Mischung aus gebackenen Brokkolisamen und Senfsaat enthält. Es wird im Dokument jedoch keinesfalls nahegelegt, dass die hierin genannten Extrakte und Drogen gemeinsam in einer Darreichungsform vorliegen können. Auch die Kapuzinerkresse wird darin nicht erwähnt.

Die US 5,882,646 A betrifft ein Nahrungsergänzungsmittel aus Brassica und ein Verfahren zu dessen Herstellung. Das Nahrungsergänzungsmittel kann Broccoli und exogene Myrosinase enthalten. Von Extrakten ist darin keine Rede, auch Kapuzinerkresse wird darin nicht erwähnt.

Die US 2020/0100532 A1 offenbart ein pulverförmiges Nahrungsmittel, das ein Gemisch aus einem mikronisiertem Pulver, das aktive Myrosinase beinhaltet, und einem Glucosinolathaltigem Pulver umfasst. Es wird im Dokument jedoch keinesfalls nahegelegt, dass die hierin genannten Extrakte und Drogen gemeinsam in einer Darreichungsform vorliegen können. Auch Meerrettich und Kapuzinerkresse werden darin nicht erwähnt.

In der US 2008/0311192 A1 wird ein Verfahren zur Umwandlung von Glucosinolat in Isothiocyanate im Dünndarm offenbart, welches die orale Verabreichung einer magensaftresistent beschichteten Vorläuferzusammensetzung eines Chemoprotektivums an ein Individuum aufweist. Es wird im Dokument jedoch keinesfalls nahegelegt, dass die hierin genannten Extrakte und Drogen gemeinsam in einer Darreichungsform vorliegen können. Auch Meerrettich und Kapuzinerkresse werden darin nicht erwähnt.

Glucosinolate können in drei Klassen unterteilt werden, die auf der Struktur der verschiedenen Aminosäurevorläufer basieren: (1) aliphatische Glucosinolate, die sich von Methionin, Isoleucin, Leucin oder Valin ableiten, (2) aromatische Glucosinolate, die sich von Phenylalanin oder Tyrosin ableiten, und (3) Indol-Glucosinolate, die sich von Tryptophan ableiten (Radojcic Redovnikovic, et al. "Glucosinolates and their potential role in plant." Periodicum biologorum 110.4 (2008): 297-309).

Im Meerrettich wurden mittlerweile 30 verschiedene Glucosinolate identifiziert (Agneta, et al. "Horseradish (Armoracia rusticana), a neglected medical and condiment species with a relevant glucosinolate profile: a review." Genetic resources and crop evolution 60.7 (2013): 1923-1943.), wobei das aliphatische Glucosinolat Sinigrin dominiert. Die Extraktkomponente (die im erfindungsgemäßen Verfahren verwendet wird bzw. in der erfindungsgemäßen Darreichungsform enthalten ist) beinhaltet somit Sinigrin. Vorzugsweise sind in der Darreichungsform (oder allein schon in der Extraktkomponente) zumindest 0,1 mg, bevorzugt zumindest 0,5 mg, mehr bevorzugt zumindest 1 mg, insbesondere zumindest 2 mg Sinigrin enthalten.

In einer besonders bevorzugten Ausführungsform sind in der Extraktkomponente (die im erfindungsgemäßen Verfahren verwendet wird bzw. in der erfindungsgemäßen Darreichungsform enthalten ist) noch zumindest ein, bevorzugt zumindest zwei, noch mehr bevorzugt zumindest drei, insbesondere zumindest vier weitere Glucosinolate enthalten, die ausgewählt sind aus der Gruppe bestehend aus: Glucoviorylin, Glucoputranjivin, Glucoarabidopsithalin, Glucoiberverin, Glucoiberin, Glucocheirolin, Glucoconringianin, Gluconapin, Glucocappariflexin, Glucoerucin, Glucokohlrabiin, Glucobrassicanapin, Glucomoracialapathin, Glucoberteroin, Glucowasabiamin, Glucolesquerellin, Gluconasturtiin, Glucoarmoracialapicin und Glucoarmoracialafolicin (vgl. Agneta et al, Tabelle 2). Insbesondere sind diese ausgewählt aus der Gruppe bestehend aus: Glucocheirolin, Gluconapin, Glucobrassicanapin und Gluconasturtiin.

Im Gegensatz dazu dominieren bei der Kapuzinerkresse aromatische Glucosinolate stark. Zu einem überwiegenden Teil macht Glucotropaeolin (Benzylglucosinolat) den Glucosinolatgehalt der Kapuzinerkresse aus, daneben ist auch Sinalbin vorhanden (Jakubczyk, et al. "Garden nasturtium (Tropaeolum majus L.)-a source of mineral elements and bioactive compounds." Roczniki Panstwowego Zakladu Higieny 69.2 (2018)). Die Extraktkomponente (die im erfindungsgemäßen Verfahren verwendet wird bzw. in der erfindungsgemäßen Darreichungsform enthalten ist) beinhaltet somit ferner Glucotropaeolin und vorzugsweise auch Sinalbin. Vorzugsweise sind in der Darreichungsform (oder allein schon in der Extraktkomponente) zumindest 0,05 mg, bevorzugt zumindest 0,1 mg, insbesondere zumindest 0,5 mg Glucotropaeolin.

Insgesamt beträgt der Glucosinolatgehalt der Darreichungsform gemäß einer bevorzugten Ausführungsform zumindest 0,15 mg, bevorzugt zumindest 0,5 mg, mehr bevorzugt zumindest 1 mg, insbesondere zumindest 2 mg. Zusätzlich oder alternativ dazu ist besonders bevorzugt, wenn die Darreichungsform (als Einzeldosis) in wässriger Lösung bei pH 1 bei einer Temperatur von 37°C innerhalb von 120 Minuten zumindest 10 pg, bevorzugt zumindest 20 pg, mehr bevorzugt zumindest 40 pg, noch mehr bevorzugt zumindest 80 $\mu$g Isothiocyanate bildet.

Das erfindungsgemäße Verfahren kann weitere Schritte umfassen, beispielsweise die gemeinsame Verpressung (z.B. zu Pellets) nach dem Trockenmischen, wodurch eine verpresste Mischung erhalten wird. Mit anderen Worten können nach dem Trockenmischen weitere Verarbeitungsschritte vorgesehen sein, um letztlich die Mischung für die pharmazeutischen Darreichungsform zu erhalten.

Zweckmäßigerweise ist das Trockenmischen ein Trockenpulvermischen, d.h. die Extraktkomponente und die Drogenkomponente liegen in diesem Fall in Pulverform vor und es werden die beiden Pulver (gegebenenfalls mit weiteren Pulvern, z.B. pulverförmigen Hilfsstoffen) miteinander vermischt.

Das Trockenmischen bringt unter anderem den Vorteil mit sich, dass die vorzeitige Umsetzung der Glucosinolate zu Isothiocyanaten durch die Myrosinase möglichst vermieden wird. Es empfiehlt sich daher auch, die Restfeuchte der trockenen Pulver bzw. die Luftfeuchtigkeit während des Trockenmischens möglichst gering zu halten.

Die trockene Extraktkomponente kann weitere Bestandteile (z.B. Extrakte aus anderen Pflanzen und/oder Hilfsstoffe) enthalten bzw. kann die Drogenkomponente weitere Bestandteile (z.B. andere pflanzliche Drogen, beispielsweise in Pulverform, und/oder Hilfsstoffe) enthalten. Bevorzugt ist jedoch, wenn die Extraktkomponente lediglich aus Meerrettich (insbesondere Meerrettichwurzel) und Kapuzinerkresse (insbesondere Kapuzinerkressenkraut) gewonnen wird.

In einer bevorzugten Ausführungsform umfasst das Bereitstellen der Extraktkomponente die Extraktion (aus Meerrettichwurzel und Kapuzinerkressenkraut sowie gegebenenfalls aus Teilen anderer Pflanzen, insbesondere der Ordnung Brassicales) mit einem Auszugsmittel. Dieses Auszugsmittel umfasst vorzugsweise ein organisches Lösungsmittel, bevorzugt Alkohol, insbesondere Methanol. Mit Methanol wurden besonders gute Ergebnisse erzielt, da das Spektrum der Inhaltsstoffe damit möglichst nah am Spektrum der Inhaltsstoffe der Rohdrogen war und zudem die Ausbeute sehr hoch war (vor allem bei 98%igem Methanol). Weitere geeignete organische Lösungsmittel sind unter anderem n-Hexan, Diethylether, Tetrahydrofuran, Dioxan und Aceton.

Das Auszugsmittel weist vorzugsweise zumindest 80% (v/v), bevorzugt zumindest 90% (v/v), mehr bevorzugt zumindest 95% (v/v), noch mehr bevorzugt zumindest 97,5% (v/v), insbesondere zumindest 98% (v/v) oder gar zumindest 99% (v/v) Alkohol auf; wobei der verbleibende Teil üblicherweise von Wasser gebildet wird. Bevorzugt ist der Alkohol Methanol oder Ethanol. Die Extraktion kann durch unterschiedliche Verfahren, wie Mazeration, Perkolation, oder Reperkolation oder andere Gegenstromverfahren erfolgen, ist aber nicht auf die genannten Verfahren limitiert. Es kann beispielsweise 5 Stunden bei 25 - 30°C extrahiert werden. Diese Extraktionszeit und Temperatur können jedoch erheblich unter- oder überschritten werden.

Um die trockene Extraktkomponente zu erhalten, kann das Auszugsmittel durch ein geeignetes Verfahren entfernt werden. Das können zum Beispiel Sprühtrocknung, oder Trocknung mittels Bandtrockner sein, aber auch andere Trocknungsverfahren können zum Einsatz kommen. Die Trocknungsparameter werden vorzugsweise so gewählt, dass das Auszugsmittel bis auf eine, in den geltenden Regularien (Ph.Eur., USP usw.) festgelegte Rückstandsmenge entfernt wird.

Es ist besonders zweckmäßig, wenn die Extraktkomponente ein Extraktpulver ist. Dieses kann nach der Extraktion mit dem Auszugsmittel erhalten werden, indem man das Auszugsmittel verdunsten lässt (z.B. in einem Rotationsverdampfer), so dass das Extraktpulver im Gefäß zurückbleibt.

Um die Rieselfähigkeit des Extraktpulvers zu verbessern bzw., je nach Extraktionsverfahren, überhaupt die Pulverform der Extraktkomponente zu erzielen (für die Füllung z.B. in Hartgelatinekapseln), können geeignete Hilfsstoffe (z.B. Maltodextrin, kolloidales Siliciumdioxid, Calciumsilikat oder entsprechende kommerziell erhältliche Silikatmischungen usw.) zugesetzt werden. Die hinzugesetzte Menge beträgt dabei vorzugsweise 30 Gew%, kann aber auch höher oder geringer sein.

Gemäß einer weiteren bevorzugten Ausführungsform ist die Extraktkomponente ein Mischextrakt aus Meerrettich (insbesondere Meerrettichwurzel) und Kapuzinerkresse (insbesondere Kapuzinerkressenkraut), und gegebenenfalls aus weiteren pflanzlichen Bestandteilen. Die Mischextraktion (d.h. die Extraktion aus einem Gemisch, wie beispielsweise einem Pulvergemisch, der beiden Drogen und gegebenenfalls weiterer pflanzlicher Drogen) bringt eine Vereinfachung des Herstellungsverfahrens mit sich, und es hat sich gezeigt, dass diese mit Meerrettichwurzel und Kapuzinerkressenkraut möglich ist, ohne dass es zu Störungen des Extraktionsprozesses (z.B. durch vorzeitigen Abbau von Produkten) kommt. Die Mischextraktion kann beispielsweise aus einer Drogenmischung im Gewichtsverhältnis Meerrettichwurzel zu Kapuzinerkressenkraut von 80 zu 200. Alternativ kann die Extraktkomponente beispielsweise auch ein (Pulver-)Gemisch aus verschiedenen (pflanzlichen) Extrakten, insbesondere aus Meerrettichwurzel und Kapuzinerkressenkraut, sein.

In einer besonders bevorzugten Ausführungsform enthält die Drogenkomponente Meerrettichwurzel und Kapuzinerkressenkraut (und gegebenenfalls weitere Bestandteile, insbesondere Hilfsstoffe). Bevorzugt besteht die Drogenkomponente aus Meerrettichwurzel und Kapuzinerkressenkraut (z.B. in Pulverform) und gegebenenfalls Hilfsstoffen (wie z.B. Füllstoffe oder Stabilisatoren); mit anderen Worten sind vorzugsweise keine weiteren pflanzlichen Drogen (oder überhaupt keine weiteren Wirkstoffe) in der Drogenkomponente enthalten.

In einer weiteren, bevorzugten Ausführungsform enthält die Drogenkomponente zumindest so viel Meerrettichwurzel und/oder Kapuzinerkressenkraut mit Myrosinase-Aktivität, dass die Mischung eine Myrosinase-Aktivität von zumindest 0,01 U/g oder gar zumindest 0,05 U/g, bevorzugt zumindest 0,1 U/g, mehr bevorzugt zumindest 0,5 U/g, noch mehr bevorzugt zumindest 1,0 U/g aufweist (bezogen auf die Trockenmasse der Drogenkomponente).

Zweckmäßigerweise liegt die Drogenkomponente in Pulverform vor.

Gemäß einer besonders bevorzugten Ausführungsform werden die Extraktkomponente und die Drogenkomponente aus den gleichen Pflanzenteilen (nämlich insbesondere Meerrettichwurzel und Kapuzinerkressenkraut) gewonnen. Dadurch wird die Qualitätskontrolle während der Herstellung vereinfacht, da keine Variation zwischen verschiedenen Batches (oder gar verschiedenen Pflanzenarten) für die Herstellung eines Batchs an Darreichungsformen berücksichtigt werden muss. Besonders bevorzugt ist dabei, wenn die Extraktkomponente aus dem gleichen Pulvergemisch (desselben Batchs) wie die Drogenkomponente gewonnen wird (mit anderen Worten: das Gewichtsverhältnis von Meerrettichwurzel und Kapuzinerkressenkraut bei der Extraktion dasselbe ist wie in der Drogenkomponente). Beispielsweise werden 1000 kg Meerrettichwurzel und 1000 kg Kapuzinerkressenkraut (jeweils bereits in Pulverform) als Ausgangsmaterial verwendet (Gewichtsverhältnis: 1:1). Diese beiden Pulver werden miteinander vermischt. Danach werden z.B. 1-10 Gew% des Pulvergemischs für die Extraktion herangezogen und z.B. mit Methanol ein pulverförmiges Mischextrakt, also die Extraktkomponente, hergestellt. Die verbliebenen 90-99 Gew% des Pulvergemischs bilden die Drogenkomponente. Schließlich werden die beiden Pulver (also das Extraktpulver und das Drogenpulver) gemeinsam beispielsweise in Kapseln (die Darreichungsform) abgefüllt.

In einer weiteren bevorzugten Ausführungsform liegt die Extraktkomponente als organischer Extrakt, bevorzugt alkoholischer Extrakt, insbesondere methanolischer oder ethanolischer Extrakt vor.

Die Darreichungsform weist in einer bevorzugten Ausführungsform (in der Drogenkomponente) eine Myrosinase-Aktivität von zumindest 0,01 U/g oder gar zumindest 0,05 U/g, bevorzugt zumindest 0,1 U/g, mehr bevorzugt zumindest 0,5 U/g, noch mehr bevorzugt zumindest 1,0 U/g (bezogen auf die Trockenmasse der Inhaltsstoffe der Darreichungsform, d.h. z.B. ohne die Eigenmasse der Kapsel bzw. des Sachets zu berücksichtigen). Gemäß einer weiteren bevorzugten Ausführungsform liegen die Extraktkomponente und/oder die Drogenkomponente in Pulverform vor. Es ist besonders vorteilhaft, wenn die Extraktkomponente und die Drogenkomponente gemeinsam in einem Pulvergemisch (z.B. in einer Kapsel) vorliegen. Dadurch wird die effiziente Umsetzung der Glucosinolate durch die Myrosinase im Gastrointestinaltrakt des Patienten nach der Einnahme gefördert.

Beispielsweise kann dieses Pulvergemisch zur Herstellung der Darreichungsform mit geeigneten Hilfsstoffen (Fließregulierungsmittel, Antiadhäsiva) durch Zumischen in einem geeigneten Mischer versetzt, und dann mit geeignetem Equipment in Hartkapseln (Hartgelatine, Cellulose usw.) gefüllt werden.

In einer weiteren, bevorzugten Ausführungsform ist die Darreichungsform eine Kapsel, eine Tablette oder ein Sachet. Wenn die Darreichungsform eine Kapsel ist, kann ihre Hülle beispielsweise im Wesentlichen aus Hartgelatine bestehen. Alternativ kann durch Anteigen der Drogen- und Extraktkomponente mit einem geeigneten Öl eine pastöse Mischung erzeugt werden, die dann in Weichgelatinekapseln gefüllt werden kann.

Gemäß einer weiteren Ausführungsform kann durch wasserfreie Granulierung (z.B Ethanol, Methanol, usw.) aus der Drogen/Extraktmischung gegebenenfalls mit Hilfstoffen ein Granulat erzeugt werden, das direkt formuliert werden kann (z.B. durch Abfüllung in Sachets) oder aber zu Tabletten verpresst werden kann. Diese Tabletten können auch mit geeigneten Hüllen überzogen werden.

Weiters könnten unter Einsatz Lösungs- oder Dispergiermittel (Wassergehalt < 4%) Liquida wie z.B. Lösungen oder Suspensionen formuliert werden, die in Flaschen oder andere geeignete Darreichungsformen (liquid pouch usw.) gefüllt werden können.

Die Darreichungsform (insbesondere die Extraktkomponente bzw. die Drogenkomponente) kann einen oder mehrere (pharmazeutisch akzeptable) Hilfsstoffe umfassen. Beispielsweise einen Stoff, ausgewählt aus der Gruppe der Polysaccharide, wie Cellulose (z.B. mikrokristalline Cellulose) Hydroxypropylmethylcellulose, Carboxymethylcellulose, Hydroxyethylcellulose, Ethylhydroxyethylcellulose, Hydroxypropyl-cellulose, Celluloseacetylphathalat, Hydroxypropylmethyl-cellulosephthalat, Celluloseacetat, Cellulosepropionat, Celluloseacetatbutyrat, Ethylcellulose, Guar-Mehl, Alginsäure und/oder Alginate, Pektin, Polyvinylpyrrolidon Polyvinylacetat, Polyvinylalkohol, Polyvinylchlorid, Polymere der Acrylsäure und deren Salze bzw. Polyacrylate bzw. Polymethacrylate bzw. Co-Polymere davon (z.B. Eudragit® E, Eudragit® R, Eudragit® S, Eudragit® NE, Eudragit® RS, Eudragit® RL), Vinylpyrrolidon-Vinylacetat-Copolymere, Nylon, kolloidale Kieselsäure (z.B. Aerosil®), Behenate, Stearate, Polyamid, Polyacrylamid, Polyethylen, Polyalkylenglykole wie Polyethylenglykol, Co-polymere von Polyalkylenglykolen, z.B. von Polyethylenglykol und Polypropylenglykol (Pluronic®, BASF), Zucker, Zuckeralkohol; und Gemischen davon. Insbesondere wenn die Darreichungsform eine Tablette ist, kann sie beispielsweise als Hilfsstoffe ein Füllmittel wie Lactose, ein Zerfallsmittel wie mikrokristalline Cellulose, ein Fließmittel wie kolloidale Kieselsäure und/oder ein Schmiermittel wie Magnesiumstearat enthalten.

In einer weiteren, bevorzugten Ausführungsform ist die Darreichungsform eine retardierte Darreichungsform (d.h. eine Darreichungsform mit verzögerter Wirkstofffreisetzung) wie z.B. eine Retardtablette. Wie eine Darreichungsform in retardierter Form formuliert werden kann, ist im Stand der Technik bekannt, beispielsweise aus der WO 2010/028794 A1 oder der WO 2010/105824 A1. Bevorzugt wird in der erfindungsgemäßen Ausführungsform ein Polymer als retardierender Hilfsstoff eingesetzt, welches Polymer ausgewählt ist aus den neutralen Homo- und Co-polymeren aus (Meth)acrylsäureestern, kationischen Homo- und Co-polymeren aus (Meth)acrylsäureestern mit quartären Ammoniumgruppen, Polyvinylacetat, Celluloseacetat, Cellulosepropionat, Celluloseacetatbutyrat, Ethylcellulose, bevorzugt aus neutralen Homo- und Co-polymeren aus (Meth)acrylsäureestern, insbesondere einem Co-Polymer aus Ethylacrylat und Methylmethacrylat.

Die Darreichungsform gemäß der vorliegenden Erfindung ist besonders gut geeignet für die Vorbeugung oder Behandlung einer Erkältungskrankheit, insbesondere eines grippalen Infekts, oder einer Infektion der Harnwege, insbesondere einer infektionsbedingten Zystitis. Bei Verabreichung an einen Patienten setzt die Darreichungsform im Gastrointestinaltrakt des Patienten vorzugsweise zumindest 1 pg, bevorzugt zumindest 5 pg, mehr bevorzugt zumindest 10 pg, insbesondere zumindest 20 oder gar zumindest 40 µg Isothiocyanate frei, um so einen prophylaktischen oder therapeutischen Effekt zu erzielen.

Für die Fachperson ist es evident, dass bei einer "trockenen" Komponente (wie einem trockenen Pulver oder Trockenpulver) bzw. beim Trockenmischen das Vorliegen einer gewissen Restfeuchte nicht ausgeschlossen ist. Bevorzugt beträgt die Restfeuchte der Extraktkomponente bzw. der Drogenkomponente (bzw. der gesamten Inhaltsstoffe der Darreichungsform gemäß der vorliegenden Erfindung) nicht mehr als 10 Gew% und insbesondere nicht mehr als 5 Gew%.

Um die Lagerstabilität zu erhöhen, ist es zweckmäßig, wenn die Darreichungsform feuchtigkeitsgeschützt gelagert wird. Daher ist in einer bevorzugten Ausführungsform die Darreichungsform verpackt in eine Coldform-Blisterpackung, eine Aluminium-Aluminium-Blisterpackung, eine High-Barrier-Kunststoff-Blisterpackung oder eine Blisterpackung mit Polyvinylidenchlorid.

Der hierin verwendete Begriff "Pulver" (wie er für sich alleine oder in Begriffen wie "Pulvergemisch", "Pulverform" oder dergleichen verwendet wird) ist breit zu verstehen und umfasst zumindest alle fließfähigen granularen Materien, insbesondere fließfähige Puder, Pulver im engeren Sinne und Granulate. Der Teilchendurchmesser kann im Median beispielsweise zwischen 1 µm und 1000 µm betragen.

Die hierin verwendete Einheit Unit (U) für die Myrosinase-Aktivität ist wie folgt definiert: 1 U Myrosinase-Aktivität ist jene Myrosinase-Aktivität, die bei 37°C 1 pmol Sinigrin in einer Minute hydrolysiert. Diese kann entweder anhand von Sinigrin-Abbau (photometrisch bestimmbar bei 230 nm) oder Glucose-Freisetzung (per gekoppeltem Enzym-Assay bestimmbar) gemessen werden (vgl. Dosz, et al. "Total myrosinase activity estimates in brassica vegetable produce." Journal of agricultural and food chemistry 62.32 (2014): 8094-8100.). Bevorzugt ist die Bestimmung der Myrosinaseaktivität über den Sinigrin-Abbau, insbesondere wie in Beispiel 3 durchgeführt.

Des Weiteren bezieht sich die vorliegende Erfindung auf die folgenden Ausführungsformen:

Ausführungsform 1. Verfahren zur Herstellung einer pharmazeutischen Darreichungsform, umfassend die folgenden Schritte:

- Bereitstellen einer trockenen Extraktkomponente aus Meerrettich (Armoracia rusticana) und Kapuzinerkresse (Tropaeolum majus) mit einer endogenen Myrosinase(EC 3.2.1.147)-Aktivität unter 0,05 U/g, bevorzugt unter 0,01 U/g, insbesondere unter 0,001 U/g;

- Bereitstellen einer trockenen Drogenkomponente mit Meerrettichwurzel (Armoraciae rusticanae radix) und/oder Kapuzinerkressenkraut (Tropaeoli maji herba);

- Trockenmischen der Extraktkomponente mit der Drogenkomponente; und

- Bereitstellen der Mischung in der pharmazeutischen Darreichungsform.

Ausführungsform 2. Verfahren gemäß Ausführungsform 1, wobei die Drogenkomponente zumindest so viel Meerrettichwurzel und/oder Kapuzinerkressenkraut mit Myrosinase-Aktivität enthält, dass die Mischung eine Myrosinase-Aktivität von zumindest 0,01 U/g oder gar zumindest 0,05 U/g, bevorzugt zumindest 0,1 U/g, mehr bevorzugt zumindest 0,5 U/g, noch mehr bevorzugt zumindest 1,0 U/g aufweist.

Ausführungsform 3. Verfahren gemäß Ausführungsform 1 oder 2, wobei die Drogenkomponente Meerrettichwurzel (Armoraciae rusticanae radix) und Kapuzinerkressenkraut (Tropaeoli maji herba) enthält.

Ausführungsform 4. Verfahren gemäß einer der Ausführungsformen 1 bis 3, wobei die Extraktkomponente ein Mischextrakt aus Meerrettich (Armoracia rusticana) und Kapuzinerkresse (Tropaeolum majus), und gegebenenfalls aus weiteren pflanzlichen Bestandteilen, ist.

Ausführungsform 5. Verfahren gemäß einer der Ausführungsformen 1 bis 4, wobei die Extraktkomponente ein Extraktpulver ist.

Ausführungsform 6. Verfahren gemäß einer der Ausführungsformen 1 bis 5, wobei das Bereitstellen der Extraktkomponente die Extraktion mit einem Auszugsmittel, umfassend ein organisches Lösungsmittel, bevorzugt Alkohol, insbesondere Methanol, umfasst.

Ausführungsform 7. Verfahren gemäß Ausführungsform 6, wobei das Auszugsmittel zumindest 80% (v/v), bevorzugt zumindest 90% (v/v), mehr bevorzugt zumindest 95% (v/v), noch mehr bevorzugt zumindest 97,5% (v/v), insbesondere zumindest 98% (v/v) oder gar zumindest 99% (v/v) Alkohol aufweist, vorzugsweise wobei der Alkohol Methanol oder Ethanol ist.

Ausführungsform 8. Verfahren gemäß einer der Ausführungsformen 1 bis 7, wobei das Trockenmischen ein Trockenpulvermischen ist.

Ausführungsform 9. Verfahren gemäß einer der Ausführungsformen 1 bis 8, wobei die Drogenkomponente in Pulverform vorliegt.

Ausführungsform 10. Verfahren gemäß einer der Ausführungsformen 1 bis 9, wobei die Extraktkomponente und die Drogenkomponente aus den gleichen Pflanzenteilen gewonnen werden.

Ausführungsform 11. Pharmazeutische Darreichungsform, herstellbar nach dem Verfahren gemäß einer der Ausführungsformen 1 bis 11.

Ausführungsform 12. Pharmazeutische Darreichungsform, umfassend:

- eine Extraktkomponente aus Meerrettich (Armoracia rusticana) und Kapuzinerkresse (Tropaeolum majus); und

- eine Drogenkomponente mit Meerrettichwurzel (Armoraciae rusticanae radix) und/oder Kapuzinerkressenkraut (Tropaeoli maji herba).

Ausführungsform 13. Pharmazeutische Darreichungsform gemäß einer der Ausführungsformen 11 und 2, wobei die Darreichungsform eine Myrosinase-Aktivität von zumindest 0,01 U/g oder gar zumindest 0,05 U/g, bevorzugt zumindest 0,1 U/g, mehr bevorzugt zumindest 0,5 U/g, noch mehr bevorzugt zumindest 1,0 U/g aufweist.

Ausführungsform 14. Pharmazeutische Darreichungsform gemäß einer der Ausführungsformen 11 bis 13, wobei die Extraktkomponente und/oder die Drogenkomponente in Pulverform vorliegen.

Ausführungsform 15. Pharmazeutische Darreichungsform gemäß einer der Ausführungsformen 11 bis 14, wobei die Extraktkomponente und die Drogenkomponente gemeinsam in einem Pulvergemisch vorliegen.

Ausführungsform 16. Pharmazeutische Darreichungsform gemäß einer der Ausführungsformen 11 bis 15, wobei die Darreichungsform eine feste Darreichungsform wie eine Kapsel, eine Tablette oder ein Sachet ist.

Ausführungsform 17. Pharmazeutische Darreichungsform gemäß einer der Ausführungsformen 11 bis 16, wobei die Darreichungsform zumindest 0,15 mg Glucosinolate enthält.

Ausführungsform 18. Pharmazeutische Darreichungsform einer der Ausführungsformen 11 bis 17, wobei die Darreichungsform in wässriger Lösung bei pH 1 bei 37°C innerhalb von 120 Minuten zumindest 10 pg, bevorzugt zumindest 20 pg, mehr bevorzugt zumindest 40 pg, noch mehr bevorzugt zumindest 80 $\mu$g Isothiocyanate bildet.

Ausführungsform 19. Pharmazeutische Darreichungsform gemäß einer der Ausführungsformen 11 bis 18, wobei die Extraktkomponente ein Mischextrakt aus Meerrettich (Armoracia rusticana) und Kapuzinerkresse (Tropaeolum majus), und gegebenenfalls aus weiteren pflanzlichen Bestandteilen, ist.

Ausführungsform 20. Pharmazeutische Darreichungsform gemäß einer der Ausführungsformen 11 bis 19, wobei die Extraktkomponente ein Extraktpulver ist.

Ausführungsform 21. Pharmazeutische Darreichungsform einer der Ausführungsformen 11 bis 20, wobei die Extraktkomponente ein organischer Extrakt, bevorzugt ein alkoholischer Extrakt, insbesondere ein methanolischer oder ethanolischer Extrakt ist.

Ausführungsform 22. Pharmazeutische Darreichungsform gemäß einer der Ausführungsformen 11 bis 21, wobei die Drogenkomponente Meerrettichwurzel (Armoraciae rusticanae radix) und Kapuzinerkressenkraut (Tropaeoli maji herba) enthält.

Ausführungsform 23. Pharmazeutische Darreichungsform gemäß einer der Ausführungsformen 11 bis 22, zur Anwendung in der Vorbeugung oder Behandlung einer Erkältungskrankheit, insbesondere eines grippalen Infekts, oder einer Infektion der Harnwege, insbesondere einer infektionsbedingten Zystitis, eines Patienten.

Ausführungsform 24. Pharmazeutische Darreichungsform zur Anwendung gemäß Ausführungsform 23, wobei die Darreichungsform im Gastrointestinaltrakt des Patientent zumindest 1 pg, bevorzugt zumindest 5 pg, mehr bevorzugt zumindest 10 pg, insbesondere zumindest 20 oder gar zumindest 40 $\mu$g Isothiocyanate freisetzt.

Im Folgenden wird die Erfindung anhand von bevorzugten, nicht einschränkenden Beispielen näher erläutert.

## Beispiel 1 - Extraktionstests

80 Teile *Armoraciae rusticanae* radix und 200 Teile *Tropaeoli maji* herba wurden auf eine zur Extraktion geeignete Partikelgröße zerkleinert (Massenverhältnis 80:200). Anschließend wurden die beiden so vorbereiteten Drogen in einem Mischer homogen vermischt. Die Myrosinaseaktivität in dieser Drogenmischung, gemessen gemäß Beispiel 3, betrug 1,0 U/g. Die Drogenmischung enthielt etwa 6000 mg/kg Sinigrin und etwa 1800 mg/kg Glucotropaeolin.
Die Extraktion wurde mit verschiedenen organischen Lösungsmitteln getestet (Extraktion: 5h bei 25 - 30°C), die Ergebnisse sind in Tabelle 1 wiedergegeben.

Tabelle 1

| Auszugsmittel | Mittelwert Ertrag Feststoffe [mg] | Resultierendes Drogen-Extraktverhältnis |
|---|---|---|
| n-Hexan | 104 | 83.3:1 |
| Diethylether | 190 | 45.6:1 |
| Tetrahydrofuran | 218 | 39.8:1 |
| Dioxan | 259 | 33.5:1 |
| Aceton | 255 | 33.9:1 |
| Ethanol 96% | 410 | 21.2:1 |
| Methanol 98% | 1201 | 7.2:1 |

Folglich stellte sich Methanol (98%) als das geeignetste Auszugsmittel für diese Drogenmischung heraus, da der Ertrag am höchsten war (und somit auch das breiteste Spektrum an verschiedensten Wirkstoffen vorlag).

**Beispiel 2** - **Herstellung der pharmazeutischen Darreichungsform**

Herstellen der Drogenkomponente

80 Teile *Armoraciae rusticanae* radix und 200 Teile *Tropaeoli maji* herba werden auf eine zur Extraktion geeignete Partikelgröße zerkleinert (Massenverhältnis 80:200). Anschließend werden die beiden so vorbereiteten Drogen in einem Mischer homogen vermischt. Die Myrosinaseaktivität in dieser Drogenmischung, gemessen gemäß Beispiel 3, beträgt 1,0 U/g. Die Drogenmischung enthält etwa 6000 mg/kg Sinigrin und etwa 1800 mg/kg Glucotropaeolin. 5 Gew% dieser Drogenmischung wird als Drogenkomponente der Darreichungsform verwendet, der verbleibende Teil einer Extraktion unterzogen (siehe nächster Absatz).

Herstellen der Extraktkomponente

In einem geeigneten Ansatzgefäss wird Methanol (>98%) vorgelegt. Die Drogenmischung wird zugefügt. Das Ansatzverhältnis beträgt 1:7 (w/w) (Drogenmischung zu Auszugsmittel), kann aber beispielsweise auch von 1:3 bis zu 1:50 oder größer betragen. Es wird 5h bei 25 - 30°C extrahiert. Nach der Extraktion wird das Auszugsmittel mittels Sprühtrocknung entfernt, um unter Zugabe von 30 Gew% Maltodextrin die trockene, pulverförmige Extraktkomponente zu erhalten. In diesem Extrakt ist keine Myrosinaseaktivität (gemessen gemäß Beispiel 3) nachweisbar. Der Extrakt enthält etwa 35000 mg/kg Sinigrin und etwa 10000 mg/kg Glucotropaeolin (ohne Berücksichtigung des Maltodextrins).

Trockenmischen

Die Drogenkomponente und die Extraktkomponente werden in einem Mischer homogen zu einem Pulvergemisch vermischt.

Abfüllen in Darreichungsform

Das Pulvergemisch wird in Hartgelatinekapseln verfüllt. Das Pulvergemisch in einer Kapsel umfasst 60 mg Drogenpulver und 150 mg Extraktpulver (ohne Berücksichtigung des Maltodextrins). Eine Kapsel (entspricht einer Einzeldosis) enthält etwa 5 mg Sinigrin und 2 mg Glucotropaeolin.

**Beispiel 3** - **Messung der Myrosinaseaktivität**

Die Myrosinase-Aktivität der Drogenmischung wird photometrisch durch den Abbau des Glucosinolats Sinigrin bestimmt. Dieser Abbau lässt sich durch die Abnahme der Absorption bei 230 nm bestimmen (Weglänge Küvette 1 cm).
Benötigte Lösungen:

- Natriumchloridlösung: 468 mg NaCl werden in 100 ml HQ Wasser gelöst = 80mM (neutraler pH-Wert)
- Substratlösung: 10 mg Sinigrin-Hydrat werden in 2 ml $H_2O$ gelöst.
- Drogenlösung: 300 mg Droge (oder Extrakt) werden in 5 ml Wasser (eiskalt) gelöst, bei 4300 rpm für 2 Minuten zentrifugiert (Kühlung der Zentrifuge auf 4°C). Anschließend wir ein Aliquot durch einen Spritzenfilter (0,45pm) filtriert.

2,97 ml der NaCl-Lösung werden in einer Küvette vorgelegt, 15 µl Substratlösung werden dazu pipettiert und durch auf- und abpipettieren gemischt.
Anschließend wird die Küvette verschlossen und in einem Wasserbad auf 37°C temperiert. In der Zwischenzeit wird die Drogenlösung hergestellt. 15 µl der Drogenlösung werden in die temperierte Küvette pipettiert und die Absorption bei 230 nm sofort gemessen. Als Blank wird eine Küvette mit reiner NaCl-Lösung verwendet. Nach Messung (T0) wird die Küvette sofort in das Wasserbad zurückgestellt und alle 5 min für einen Zeitraum von 30 min gemessen.
Die Enzymaktivität wird über die Glucosinolat-Abnahme über 30 min bestimmt:

$$\text{Myrosinase activity} = \frac{1000 \times V_A}{(V_{sample} \times C)} \times \frac{2 \times (A_0 - A_t)}{E \times t}$$

$V_A$       Volumen der Reaktionsmixtur in ml (3)

$A_0$          Absorption bei Messbeginn

$A_t$          Absorption nach Zeit t

t          Reaktionszeit in min (30)

$V_{sample}$          Volumen der untersuchten Probe in ml (0,015)

C          Konzentration der Probe in g/ml (0,300/5)

E          molarer Extinktionskoeffizient für Sinigrin (7500 $M^{-1}$ $cm^{-1}$)

Die Myrosinaseaktivität in Unit gibt pmol Glucosinolate (Sinigrin) an, die pro Minute hydrolysiert werden. Kalkuliert auf 1 g Droge (oder Extrakt) ergibt das eine Enzymaktivität von U/g Droge (oder Extrakt).

**Beispiel 4** - **Freisetzungsverhalten der Darreichungsform**

Das gemäß Beispiel 1 hergestellte Pulvergemisch entsprechend einer Einzeldosis (d.h. der Inhalt einer Kapsel) wurde hinsichtlich des Isothiocyanat-Freisetzungsverhaltens untersucht.

Um die Bedingungen im Gastrointestinaltrakt zu simulieren, wurde eine allgemein anerkannte Methode auf der Grundlage der Pharmacopoea Europaea verwendet. Das Pulvergemisch wurde in 100 ml Wasser bei 37°/pH 1 (Salzsäure) dispergiert und gerührt. Nach 0 (Kontrolle), 2 und 3 Stunden wurden Proben entnommen und auf den Gehalt an Allylisothiocyanat untersucht. Nach 2 Stunden wurde der pH-Wert mit Natriumhydroxid auf 7 eingestellt, und die Mischung wurde weiter gerührt.

Nach 2 Stunden wurden aus dieser Einzeldosis hochgerechnet ca. 120 μg Allylisothiocyanat freigesetzt, dieser Wert hatte sich am Messpunkt 3h nicht mehr wesentlich verändert.

**Patentansprüche**

1. Verfahren zur Herstellung einer pharmazeutischen Darreichungsform, umfassend die folgenden Schritte:

   - Bereitstellen einer trockenen Extraktkomponente aus Meerrettich (*Armoracia rusticana*) und Kapuzinerkresse (*Tropaeolum majus*) mit einer endogenen Myrosinase(EC 3.2.1.147)-Aktivität unter 0,05 U/g, bevorzugt unter 0,01 U/g, insbesondere unter 0,001 U/g;
   - Bereitstellen einer trockenen Drogenkomponente mit Meerrettichwurzel (*Armoraciae rusticanae* radix) und/oder Kapuzinerkressenkraut (*Tropaeoli maji* herba);
   - Trockenmischen der Extraktkomponente mit der Drogenkomponente; und
   - Bereitstellen der Mischung in der pharmazeutischen Darreichungsform.

2. Verfahren gemäß Anspruch 1, wobei die Drogenkomponente zumindest so viel Meerrettichwurzel und/oder Kapuzinerkressenkraut mit Myrosinase-Aktivität enthält, dass die Mischung eine Myrosinase-Aktivität von zumindest 0,01 U/g oder gar zumindest 0,05 U/g, bevorzugt zumindest 0,1 U/g, mehr bevorzugt zumindest 0,5 U/g, noch mehr bevorzugt zumindest 1,0 U/g aufweist.

3. Verfahren gemäß Anspruch 1 oder 2, wobei die Drogenkomponente Meerrettichwurzel (*Armoraciae rusticanae* radix) und Kapuzinerkressenkraut (*Tropaeoli maji* herba) enthält.

4. Verfahren gemäß einem der Ansprüche 1 bis 3, wobei die Extraktkomponente ein Mischextrakt aus Meerrettich (*Armoracia rusticana*) und Kapuzinerkresse (*Tropaeolum majus*), und gegebenenfalls aus weiteren pflanzlichen Bestandteilen, ist.

5. Verfahren gemäß einem der Ansprüche 1 bis 4, wobei das Bereitstellen der Extraktkomponente die Extraktion mit einem Auszugsmittel, umfassend ein organisches Lösungsmittel, bevorzugt Alkohol, insbesondere Methanol, umfasst.

6. Verfahren gemäß Anspruch 5, wobei das Auszugsmittel zumindest 80% (v/v), bevorzugt zumindest 90% (v/v), mehr bevorzugt zumindest 95% (v/v), noch mehr bevorzugt zumindest 97,5% (v/v), insbesondere zumindest 98% (v/v) oder gar zumindest 99% (v/v) Alkohol ist, vorzugsweise wobei der Alkohol Methanol oder Ethanol ist.

7. Verfahren gemäß einem der Ansprüche 1 bis 6, wobei die Extraktkomponente und die Drogenkomponente aus den gleichen Pflanzenteilen gewonnen werden.

8. Pharmazeutische Darreichungsform, herstellbar nach dem Verfahren gemäß einem der Ansprüche 1 bis 7.

9. Pharmazeutische Darreichungsform, umfassend:

    - eine Extraktkomponente aus Meerrettich (*Armoracia rusticana*) und Kapuzinerkresse (*Tropaeolum majus*); und
    - eine Drogenkomponente mit Meerrettichwurzel (*Armoraciae rusticanae* radix) und/oder Kapuzinerkressenkraut (*Tropaeoli maji* herba).

10. Pharmazeutische Darreichungsform gemäß Anspruch 8 oder 9, wobei die Darreichungsform eine Myrosinase-Aktivität von zumindest 0,01 U/g oder gar zumindest 0,05 U/g, bevorzugt zumindest 0,1 U/g, mehr bevorzugt zumindest 0,5 U/g, noch mehr bevorzugt zumindest 1,0 U/g aufweist.

11. Pharmazeutische Darreichungsform gemäß einem der Ansprüche 8 bis 10, wobei die Extraktkomponente und die Drogenkomponente gemeinsam in einem Pulvergemisch vorliegen.

12. Pharmazeutische Darreichungsform gemäß einem der Ansprüche 8 bis 11, wobei die Darreichungsform eine feste Darreichungsform wie eine Kapsel, eine Tablette oder ein Sachet ist.

13. Pharmazeutische Darreichungsform gemäß einem der Ansprüche 8 bis 12, wobei die Darreichungsform zumindest 0,15 mg Glucosinolate enthält.

14. Pharmazeutische Darreichungsform gemäß einem der Ansprüche 8 bis 13, wobei die Darreichungsform in wässriger Lösung bei pH 1 bei 37°C innerhalb von 120 Minuten zumindest 10 pg, bevorzugt zumindest 20 pg, mehr bevorzugt zumindest 40 pg, noch mehr bevorzugt zumindest 80 $\mu$g Isothiocyanate bildet.

15. Pharmazeutische Darreichungsform gemäß einem der Ansprüche 8 bis 14, wobei die Extraktkomponente ein organischer Extrakt, bevorzugt ein alkoholischer Extrakt, insbesondere ein methanolischer oder ethanolischer Extrakt ist.

Europäisches
Patentamt

European
Patent Office

Office européen
des brevets

**EUROPÄISCHER RECHERCHENBERICHT**

Nummer der Anmeldung

EP 23 17 4069

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | US 6 824 796 B2 (ACCESS BUSINESS GROUP INT LLC [US]) 30. November 2004 (2004-11-30) * Spalte 2, Zeilen 35-41; Ansprüche * ----- | 1-15 | INV. A61K36/185 A61K36/31 A61K9/14 A61P31/00 |
| Y,D | US 2013/072554 A1 (ZHANG YUESHENG [US] ET AL) 21. März 2013 (2013-03-21) * Absatz [0006]; Ansprüche * ----- | 1-15 | |
| Y,D | US 2010/317518 A1 (STEVENS JAN F [US] ET AL) 16. Dezember 2010 (2010-12-16) * Ansprüche * ----- | 1-15 | |
| Y,D | US 2020/100532 A1 (DANDUKA KOHEI [JP] ET AL) 2. April 2020 (2020-04-02) * Absatz [0004]; Ansprüche * ----- | 1-15 | |
| Y | CONRAD ANDREAS ET AL: "In vitro study to evaluate the antibacterial activity of a combination of the haulm of nasturtium (Tropaeoli majoris herba) and of the roots of horseradish (Armoraciae rusticanae radix)", ARZNEIMITTEL-FORSCHUNG DRUG RESEARCH, ECD EDITTIO CANTOR VERLAG , AULENDORF, DE, Bd. 56, Nr. 12, 1. Januar 2006 (2006-01-01), Seiten 842-849, XP001525756, ISSN: 0004-4172 * das ganze Dokument * ----- | 1-15 | RECHERCHIERTE SACHGEBIETE (IPC) A61P A61K |
| Y,D | BR PI0 801 686 A2 (KRAFT FOODS HOLDINGS INC [US]) 26. Januar 2010 (2010-01-26) * Ansprüche * ----- | 1-15 | |

Der vorliegende Recherchenbericht wurde für alle Patentansprüche erstellt

| Recherchenort | Abschlußdatum der Recherche | Prüfer |
|---|---|---|
| München | 23. Oktober 2023 | Langer, Astrid |

KATEGORIE DER GENANNTEN DOKUMENTE

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus anderen Gründen angeführtes Dokument

................................................................
& : Mitglied der gleichen Patentfamilie, übereinstimmendes
   Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 23 17 4069

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

23-10-2023

| Im Recherchenbericht angeführtes Patentdokument | Datum der Veröffentlichung | Mitglied(er) der Patentfamilie | Datum der Veröffentlichung |
|---|---|---|---|
| US 6824796 B2 | 30-11-2004 | KEINE | |
| US 2013072554 A1 | 21-03-2013 | CA 2791988 A1 | 15-09-2011 |
| | | CN 102905713 A | 30-01-2013 |
| | | EP 2544699 A2 | 16-01-2013 |
| | | US 2013072554 A1 | 21-03-2013 |
| | | WO 2011112919 A2 | 15-09-2011 |
| US 2010317518 A1 | 16-12-2010 | AU 2008275894 A1 | 22-01-2009 |
| | | CA 2693262 A1 | 22-01-2009 |
| | | NZ 581988 A | 29-06-2012 |
| | | US 2010317518 A1 | 16-12-2010 |
| | | WO 2009012485 A1 | 22-01-2009 |
| US 2020100532 A1 | 02-04-2020 | CN 110831445 A | 21-02-2020 |
| | | JP 7057070 B2 | 19-04-2022 |
| | | JP 2019004709 A | 17-01-2019 |
| | | US 2020100532 A1 | 02-04-2020 |
| | | WO 2018235805 A1 | 27-12-2018 |
| BR PI0801686 A2 | 26-01-2010 | AR 066965 A1 | 23-09-2009 |
| | | AU 2008202521 A1 | 08-01-2009 |
| | | BR PI0801686 A2 | 26-01-2010 |
| | | CA 2634785 A1 | 12-12-2008 |
| | | CN 101332295 A | 31-12-2008 |
| | | EP 2065034 A2 | 03-06-2009 |
| | | NZ 568974 A | 27-11-2009 |
| | | US 2008311192 A1 | 18-12-2008 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2009012485 A1
- US 9486434 B2
- US 5882646 A
- US 20200100532 A1
- US 20080311192 A1
- WO 2010028794 A1
- WO 2010105824 A1

**In der Beschreibung aufgeführte Nicht-Patentliteratur**

- **NEGRO et al.** Phytochemical and functional analysis of horseradish (Armoracia rusticana) fermented and non-fermented root extracts. *Fitoterapia,* 2022, vol. 162, 105282
- **VRCA et al.** Chemical Composition and Biological Activity of Essential Oil and Extract from the Seeds of Tropaeolum majus L. var. altum. *Food Technology and Biotechnology,* 2022, vol. 60, 4
- **RADOJCIC REDOVNIKOVIC et al.** Glucosinolates and their potential role in plant. *Periodicum biologorum,* 2008, vol. 110.4, 297-309
- **AGNETA et al.** Horseradish (Armoracia rusticana), a neglected medical and condiment species with a relevant glucosinolate profile: a review. *Genetic resources and crop evolution,* 2013, vol. 60.7, 1923-1943
- **JAKUBCZYK et al.** Garden nasturtium (Tropaeolum majus L.)-a source of mineral elements and bioactive compounds. *Roczniki Panstwowego Zakladu Higieny,* 2018, vol. 69.2
- **DOSZ et al.** Total myrosinase activity estimates in brassica vegetable produce. *Journal of agricultural and food chemistry,* 2014, vol. 62.32, 8094-8100